# EUROPEAN PATENT SPECIFICATION

(11) **EP 1 337 267 B1**
(45) Date of publication and mention of the grant of the patent: **25.04.2007**
(21) Application number: 01992584.1
(22) Date of filing: 05.11.2001
(51) Int. Cl.: A61K 38/45, A61P 7/04, A61K 38/37, A61K 38/11

(54) **USE OF BLOOD COAGULATION FACTOR XIII FOR TREATING HAEMOPHILIA A**
VERWENDUNG DES BLUTGERINNUNGSFAKTORS XIII FÜR DIE BEHANDLUNG DER HÄMOPHILIE A
UTILISATION DU FACTEUR XIII DE COAGULATION SANGUINE DANS LE TRAITEMENT DE L'HEMOPHILIE A

(30) Priority: 03.11.2000 US 245751 P
(43) Date of publication of application: 27.08.2003
(73) Proprietor: ZymoGenetics, Inc., Seattle, WA 98102 (US)
(72) Inventor: ÖHRSTRÖM, Jan, Mercer Island, WA 98040 (US); BISHOP, Paul, D., Fall City, WA 98024 (US); ROSE, Lynn, Massman, Seattle, WA 98114 (US)
(74) Representative: MacLean, Martin Robert
(86) International application number: PCT/US2001/047073
(87) International publication number: WO 2002/036155

(56) References cited:
- EP-A- 0 268 772
- WO-A-00/27409
- WO-A-01/85198
- WO-A-89/01512
- EDITORS: BEERS-MH AND BERKOW-R: "The Merck Manual of Diagnosis and Therapy - 17th Edition" 1999 , MERCK RESEARCH LABORATORIES XP002191646 page 888, right-hand column, last paragraph
- BERRY-EW: "Use of DDAVP and cryoprecipitate in mild to moderate haemophilia A and von Willebrand's disease" PROG CLIN BIOL RES, vol. 324, 1990, pages 269-278, XP001064004
- KARGES-HE ET AL.: "Therapeutic factor XIII preparations and perspectives for recombinant factor XIII" SEMIN THROMB HEMOST, vol. 22, no. 5, 1996, pages 427-436, XP001064015

## Description

Hemophilia A is an inherited disorder of blood coagulation characterized by a permanent tendency to hemorrhage due to a defect in the blood coagulation mechanism. Hemophilia A is caused by a deficiency in factor VIII. Factor VIII coagulant protein is a single-chain protein that regulates the activation of factor X by proteases in the intrinsic coagulation pathway. It is synthesized in liver parenchymal cells and circulates complexed to the von Willebrand protein. One in 10,000 males is born with deficiency or dysfunction of the factor VIII molecule. The resulting disorder, hemophilia A is characterized by bleeding into soft tissues, muscles, and weight-bearing joints. Although normal hemostasis requires 25 percent factor VIII activity, symptomatic patients usually have factor VIII levels below 5 percent.

Hemophilic bleeding occurs hours or days after injury, can involve any organ and, if untreated, may continue for days or weeks. This can result in large collections of partially clotted blood putting pressure on adjacent normal tissues and can cause necrosis of muscle, venous congestion, or ischemic damage to nerves. Hemophilia A is generally treated by administering to the patient either recombinant or plasma-derived factor VIII, or mild cases can be treated with desmopressin. However, there are times when treating such patients with factor VIII or desmopressin produces less than satisfactory results, and hemorrhaging continues.

Thus, there is a need to develop additional therapies for treating hemophilia A.

Merck Manual 1999, page 888, right-hand column, last paragraph describes cryoprecipitated antihaemophilic factor (factor VIII).

Prog. Clin. Biol. Res. 1990, Vol 324, pages 269-278, describes the use of DDAVP and cryoprecipitate in mild to moderate haemophilia A.

WO 00/27409 describes the production of biological factors and creation of an immunologically privileged environment using genetically altered Sertoli cells.

Seminars in Thrombosis and Hemostasis 1996, 22(5), pages 427-436, relates to expression and physicochemical properties of recombinant factor XIII.

EP 0,268,772 relates to the expression of biologically active factor XIII.

WO 01/85198 describes a pharmaceutical composition comprising a factor VIIa and a factor XIII.

The present invention provides the use of recombinant factor XIII as the sole pharmaceutically active component for the production of a medicament for the treatment of haemophilia A.

The present invention thus fills the above-described need in the art by administering recombinant factor XIII to patients with haemophilia A, preferably in conjunction with either factor VIII or desmopressin acetate, or both factor VIII and desmopressin acetate.

### Diagnosis of Hemophilia A

Once a bleeding disorder has been determined to be present, the physician must determine what is the cause of the disorder. For diagnostic purposes, the hemostatic system is divided into two parts: the plasma coagulation factors, and platelets. With the exception of factor XIII deficiency, each of the known defects in coagulation proteins prolongs either the prothrombin time (PT), partial thromboplastin time (PTT), or both of these laboratory screening assays. A PT is performed by addition of a crude preparation of tissue factor (commonly an extract of brain) to citrate-anticoagulated plasma, recalcification of the plasma, and measurement of the clotting time. A PTT assay is performed by the addition of a surface activating agent, such as kaolin, silica, or ellagic acid, and phospholipid to citrate-anticoagulated plama. After incubation for a period sufficient to provide for the optimal activation of the contact factors, the plasma is recalcified and the clotting time measured. The name of the PTT assay emanates from the phospholipid reagents being originally derived from a lipid-enriched extract of complete thromboplastin, hence the term partial thromboplastin. The PTT assay is dependent on factors of both the intrinsic and common pathways. The PTT may be prolonged due to a deficiency of one or more of these factors or to the presence of inhibitors that affect their function. Although its commonly stated that decreases in factor levels to approximately 30% of normal are required to prolong the PTT, in practice the variability is considerable in sensitivity of different commercially available PTT reagents to the various factors. In fact, the levels may vary from 25% to 40%. See, Miale JB: *Laboratory Medicine-Hematology*. 6^{th} Ed., (CV Mosby, St. Louis, MS, 1982). If the PT and PTT are abnormal, quantitative assays of specific coagulation proteins are then carried out using the PT or PTT tests and plasma from congenitally deficient individuals as substrate. The corrective effect of varying concentration of patient plasma is measured and expressed as a percentage of normal pooled plasma standard. The interval range for most coagulation factors is from 50 to 150 percent of this average value, and the minimal level of most individual factors needed for adequate hemostasis is 25 percent.

### Factor VIII

The specific activity of pure factor VIII ranges from 2,300 U/mg to 8,000 U/mg. For standardization, 1U of factor VIII is defined as that amount of activity in 1 ml of normal pooled human plasma measured in a factor VIII assay by using factor VIII-deficient plasma. The frequency and severity of bleeding in hemophilia A may be predicted from the factor VIII procoagulant level assayed in comparison to a reference standard that is assumed to have factor VIII levels of 100%, corresponding to a factor VIII activity of 1.0 U/ml of plasma. The factor VIII level in normal persons ranges from 0.50 -2.0 U/ml. Those with factor VIII levels < 1% of normal (<0.1 U/ml) have hemorrhages requiring therapy two to four times a month on average. Such patients are classified as severe hemophiliacs. Those with factor VIII levels >5% of normal (>0.05 U/ml) are considered mild hemophiliacs and usually hemorrhage only due to trauma or surgery.

### Treatment of Hemophilia A

The hemostatically effective plasma level for each coagulation factor is different and depends in part on the nature, extent, and duration of the bleeding lesion. The dose of replacement factor is calculated in units: 1 U is the activity of a given coagulation factor found in 1 ml of pooled, citrated fresh frozen human plasma. The factor must be given in sufficient quantity to allow for its clearance, metabolic half-life, and volume of distribution within the body.

The half-life of factor VIII in plasma is between 8 and 12 hours, which includes an initial rapid decline in level owing to diffusion into extravascular pools. The minimum hemostatic level of factor VIII for relatively mild hemorrhages is 30% (0.3 U/ml of plasma), while that for advanced joint or muscle bleeding or for other major hemorrhagic lesions is 50% (0.50 U/ml of plasma). One to several days of maintenance therapy is needed for such advanced lesions to heal. Resolution is generally achieved by repeating the infusion at 24-hour intervals at approximately 75% of the original dose. For life-threatening lesions or surgery, levels of 80%-100% (0.8-1.00 U/ml of plasma) should be achieved and the factor VIII level should be kept above the 30%-50% range by means of appropriate doses of factor VIII infused at intervals of 8-12 hours. This more frequent infusion regimen decreases the incidence of excessively low levels of factor VIII just prior to an infusion and also decreases the total amount of factor needed to maintain given *in vivo* minimum plasma levels. Constant infusion regimens are another option when levels need to be maintained above a set minimum.

Doses can be calculated by multiplying the recipient plasma volume in milliliters by the desired increment of factor VIII in units per milliliter. A simpler and reproducible dose calculation is that each unit of factor VIII infused per kilogram of body weight yields a 2% rise in plasma factor VIII level (i.e., 0.02 U/ml of plasma). An example of therapy for a 50-kg patient with an extensive laceration would include maintenance of a 30% factor VIII level *in vivo* until healing is complete. This can be accomplished by an initial infusion to the 60% level with 1500 U (30 x 50 kg) of factor VIII, followed by 750 U every 12 hours thereafter for 7-10 days, with dose adjustments being made every few days as indicated by factor VIII assays.

### Treatment of Hemophilia A with Factor VIII and Factor XIII

The method of the present invention improves upon the above-described treatment of hemophilia A by administering factor XIII in conjunction with factor VIII. The factor XIII can be administered at any time alone or at the same time as factor VIII either to stop a hemorrhage or for prophylaxis.

Factor XIII, also known as fibrin-stabilizing factor, circulates in the plasma at a concentration of 20 µg/ml. The protein exists in plasma as a tetramer comprised of two A subunits and two B subunits. Each subunit has a molecular weight of 83,000 Da, and the complete protein has a molecular weight of approximately 330,000 Da. Factor XIII catalyzes the cross-linkage between the γ-glutamyl and ε-lysyl groups of different fibrin strands. The catalytic activity of factor XIII resides in the A subunits. The B subunits act as carriers for the A subunits in plasma factor XIII. The level of factor XIII in the plasma can be increased by administering a factor XIII concentrate derived from human placenta called FIBROGAMMIN® (Aventis Corp.) or by administration of recombinant factor XIII.. Recombinant factor XIII can be produced according to the process described in European Patent No. 0 268 772 B1.

A pharmaceutical composition comprising factor XIII can be formulated according to known methods to prepare pharmaceutically useful compositions, whereby the therapeutic proteins are combined in a mixture with a pharmaceutically acceptable carrier. A composition is said to be a "pharmaceutically acceptable carrier" if its administration can be tolerated by a recipient patient. A suitable pharmaceutical composition of factor XIII will contain 1mM EDTA, 10mM Glycine, 2% sucrose in water. An alternative formulation will be a factor XIII composition containing 20 mM histidine, 3% wt/volume sucrose, 2 mM glycine and .01% wt/vol. polysorbate, pH 8. The concentration of factor XIII should preferably be 1 - 10 mg/mL, more preferably about 5 mg/mL.

Other suitable carriers are well known to those in the art. See, for example, Gennaro (ed.), *Remington's Pharmaceutical Sciences,* 19th Edition (Mack Publishing Company 1995).

### Administration of Factor XIII

Factor XIII can be administered intravenously, intramuscularly or subcutaneously to treat hemophilia A. When administering therapeutic proteins by injection, the administration may be by continuous infusion or by single or multiple boluses. The levels of factor XIII in an individual can be determined by assays well known in the art such as the BERICHROM^{®} F XIII assay (Dade Behring Marburgh GmbH, Marburg, Germany). The normal adult has an average of about 45 ml of plasma per kg of body weight. Each liter of blood has 1000 units (U) of factor XIII. The amount of factor XIII administered should be enough to bring an individual's level of factor XIII in the plasma to 100% of normal plasma or slightly above to 1-5% above normal. A dose of .45 U/kg would raise the level of factor XIII by about 1% compared to normal. One unit of factor XIII is about 10 µg of recombinant factor XIII, which contains only the dimerized A subunit. Thus, to raise the level of factor XIII by 1%, one would administer about 4.5 µg of the A2 subunit per kilogram weight of the individual. So to raise the level 30% of normal, one would administer 13.5 U/kg. For a 75 kg individual this would be about 1,012.5 U. Some patients may have consumptive coagulopathies that involve factor XIII losses. In such cases, a higher dosing (*e.g.,* 1-2U/kg-%) or multiple dosing of factor XIII (*e.g.,* 1-2U/kg-%-day) may be required.

### Treatment of Mild hemophilia A with Desmopressin

Patients with mild hemophilia A (factor VIII levels >5% of normal) do not bleed spontaneously, but usually only after trauma or surgical procedures. The current treatment of choice for patients with factor VIII levels >10% is desmopressin. Desmopressin is a synthetic analogue of the natural pituitary hormone 8-arginine vasopressin. Although its exact mechanism is not known, it is thought to stimulate release of factor VIII from storage sites. The routine dosage is a 0.3 ?g/kg in 50 ml of normal saline given intravenously over a period of 30-40 minutes. To assess how an individual patient will respond to desmopressin, a staging test should be done. When the patient is not bleeding, a baseline factor VIII level is obtained and then the dose of desmopressin is administered. Thirty to 45 minutes after the infusion stops, a second factor VIII level is checked. The factor VIII level should rise at least threefold. If the levels rise to >80% of normal, the response is adequate for major surgery. In some patients the response desmopressin can only be used for minor hemorrhages since the factor VIII levels do not rise sufficiently. Desmopressin can also be administered with factor VIII if needed. When desmopressin is used for major surgery, it should be given 1 hour before surgery and then every 12 hours. Tachyphylaxis may occur after repeated doses of desmopressin secondary to depletion of factor VIII from storage sites. Thus factor VIII levels frequently after the first two days of administration of desmopressin. If tachyphylaxis does occur, factor VIII should be administered.

### Treatment of Mild Hemophilia with Desmopressin and Factor XIII

The present invention also encompasses administering factor XIII in conjunction with desmopressin to treat hemophilia A. The administration of factor XIII with desmopressin may even prevent the tachyphylaxis described above.

Factor VIII is produced by a number of companies in both a recombinant and plasma-derived formulations. Among these are the following: KOGENATE® (a recombinant factor VIII) produced by Bayer Corp. West Haven, CT; RECOMBINATE® (a recombinant factor VIII) produced by Baxter Healthcare Corp., Glendale, CA; HELIXATE® (a recombinant factor VIII) produced by Centeon L.L.C., King of Prussia, PA; HEMAFIL M (human, plasma-derived) produced by Baxter Healthcare Corp.; HUMATE-P CONCENTRATE® (human, plasma-derived) produced by Centeon L.L.C.; KOATE-DVI® (human, plasma-derived) produced by Bayer Biological; KOATE HP (human, plasma-derived) produced by Bayer Biological; MONOCLATE-P® (human, plasma-derived) produced by Centeon L.L.C.

Desmopressin acetate is produced by Rhône-Poulenc Rorer, Collegeville, PA, by Ferring Pharmaceutical, Tarrytown, NY, and by Centeon, King of Prussia PA.

## Claims

1. The use of recombinant factor XIII as the sole pharmaceutically active component for the production of a medicament for the treatment of hemophilia A.

2. Use according to Claim 1 wherein said factor XIII is administered at a level of .45 units per kilogram weight of an individual for every 1% rise in plasma factor XIII levels desired.

3. Use according to Claim 1 or 2 wherein the factor XIII is administered at the time of a bleeding episode.

4. The use of recombinant factor XIII A2 dimer in conjunction with factor VIII and/or desmopressin for the production of a medicament for the treatment of hemophilia A.

5. Use according to Claim 4 wherein said recombinant factor XIII A2 dimer is administered at a level of .45 units per kilogram weight of an individual for every 1% rise in plasma factor XIII levels desired.

## Patentansprüche

1. Verwendung des rekombinanten Faktors XIII als einzige pharmazeutisch aktive Komponente zur Herstellung eines Medikaments zur Behandlung von Hämophilie A.

2. Verwendung gemäß Anspruch 1, wobei der Faktor XIII bei einer Konzentration von 0,45 Einheiten ("units") pro Kilogramm Gewicht eines Individuums für jeden gewünschten Anstieg um 1% der Konzentrationen des Faktors XIII im Plasma verabreicht wird.

3. Verwendung gemäß Anspruch 1 oder 2, wobei der Faktor XIII zur Zeit einer Blutung verabreicht wird.

4. Verwendung des rekombinanten Faktor-XIII-A2-Dimers in Verbindung mit Faktor VIII und/oder Desmopressin zur Herstellung eines Medikaments zur Behandlung von Hämophilie A.

5. Verwendung gemäß Anspruch 4, wobei das rekombinante Faktor-XIII-A2-Dimer bei einer Konzentration von 0,45 Einheiten ("units") pro Kilogramm Gewicht eines Individuums für jeden gewünschten Anstieg um 1% der Konzentrationen des Faktors XIII im Plasma verabreicht wird.

## Revendications

1. Utilisation du facteur XIII recombinant en tant que seul composant pharmaceutiquement actif pour la fabrication d'un médicament destiné au traitement de l'hémophilie A.

2. Utilisation selon la revendication 1, dans laquelle ledit facteur XIII est administré à un taux de 0,45 unité par kilogramme de poids d'un individu pour chaque augmentation de 1% du taux plasmatique de facteur XIII désirée.

3. Utilisation selon la revendication 1 ou 2, dans laquelle le facteur XIII est administré au moment d'un épisode de saignement.

4. Utilisation d'un dimère A2 du facteur XIII recombinant en association avec le facteur VIII et/ou la desmopressine pour la fabrication d'un médicament destiné au traitement de l'hémophilie A.

5. Utilisation selon la revendication 4, dans laquelle ledit dimère A2 du facteur XIII recombinant est administré à un taux de 0,45 unité par kilogramme de poids d'un individu pour chaque augmentation de 1% du taux plasmatique de facteur XIII désirée.
